Europäisches Patentamt

European Patent Office

Office européen des brevets

(19)

(11) Numéro de publication: **0 068 964**
**B1**

(12) **FASCICULE DE BREVET EUROPEEN**

(45) Date de publication du fascicule du brevet:
19.03.86

(51) Int. Cl.⁴: **A 61 F 5/44**

(21) Numéro de dépôt: **82401077.1**

(22) Date de dépôt: **14.06.82**

(54) Système de filtre et d'évent intégré dans des poches de drainage d'anus artificiel, et poches de drainage ainsi équipées.

(30) Priorité: **15.06.81 FR 8111757**

(43) Date de publication de la demande:
**05.01.83 Bulletin 83/1**

(45) Mention de la délivrance du brevet:
**19.03.86 Bulletin 86/12**

(73) Titulaire: **LABORATOIRES BIOTROL S.A., 1, rue du Foin, F-75140 Paris Cedex 03 (FR)**

(72) Inventeur: **Jacolin, Pierre, 15, rue Marcel Renault, F-75017 Paris (FR)**

(74) Mandataire: **Phélip, Bruno et al, c/o Cabinet Harlé & Phélip 21, rue de la Rochefoucauld, F-75009 Paris (FR)**

(84) Etats contractants désignés:
**AT BE CH DE GB IT LI NL SE**

(56) Documents cités:
**FR - A - 2 310 739**
**FR - A - 2 431 285**
**FR - A - 2 443 832**
**US - A - 4 211 224**

LIBER, STOCKHOLM 1986

## Description

La présente invention concerne une poches de drainage d'anus artificiel, et plus particulièrement un système original de filtre et d'évent intégré dans de telles poches.

Les patients ayant subi une opération chirurgicale telle qu'une colostomie ou une iléostomie avec création d'anus artificiel sont généralement équipés de poches de recueil réalisées en matériau souple, notamment en matière plastique, composées de feuilles ou films de ce matériau imperméables aux gaz et aux odeurs, soudées entre elles sur leur périphérie et dont l'une comporte une ouverture frontale appropriée pour assurer une communication et une liaison pratiquement étanche entre l'anus artificiel et l'intérieur de la poche.

Les excrétions solides ou liquides émises et recueillies dans cette poche s'accompagnent de l'émission de gaz intestinaux; l'évacuation des gaz contenus dans la poche est nécessaire pour éviter que celle-ci gonfle excessivement; cependant le maintien d'un minimum de pression résiduelle est vivement souhaité, pour un meilleur confort du patient et surtout pour éviter que les deux films de la poche ""collapsent", c'est-à-dire se collent l'un à 1'-autre, formant un étranglement préjudiciable à une utilisation convenable de la poche. D'autre part, certains gaz intestinaux dégagent une odeur qui est genante pour le patient et son entourage et il s'est donc très vite avéré avantageux de munir les poches de drainage d'anus artificiel d'un filtre placé sur le trajet des gaz de telle sorte que ceux-ci doivent le traverser avant de sortir de la poche.

Les filtres actuellement utilisés sont soit fixés axérieurement ou intérieurement sur 1'-un des deux films de la poche avant la réalisation de celle-ci, soit rapportés sur la face externe de l'un des deux films de la poche fabriquée préalablement.

Dans l'un comme dans l'autre de ces deux cas, des variantes ont été élaborées dans le but de rendre plus efficaces de tels filtres, tout en leur conservant un volume limité. Pour certaines de ces variantes, il s'est agi de faire dévier radialement les gaz à l'intérieur de la pastille de filtre dans laquelle ils ont pénétré soit par la périphérie, soit par un trou central, ou encore de faire suivre au gaz un parcours complexe par un jeu de déviations et de chicanes à l'intérieur de la pastille de filtre qui a la forme d'un disque ou d'un anneau. A titre de documents illustrant l'état de la technique brièvement résumé ci-dessus, on peut citer les demandes de brevet français publiées sous les n° 2.443.832, 2.310.739 et 2.431.285. Un autre brevet antérieur est le brevet US 4.211.224 qui décrit un dispositif de dégazage de poche associé à (ou intégré dans) la partie haute de la ligne de soudure de la poche. Il comprend un tube de matière plastique dans lequel s'insère exactement la cartouche filtrante. Il comporte aussi un agent désodorisant volatil. Il ne comporte pas de ligne de soudure autre que celle nécessaire à la fermeture normale de la poche. Dans une variante de réalisation, ce dispositif peut comporter une partie en forme de chambre évitant que l'agent désodorisant volatil s'évapore. Une telle partie, si elle est présente, est totalement indépendante de la structure des soudures des films de la poche. Elle doit d'ailleurs être réalisée avant les soudures. Dans la pratique, le filtre doit toujours rester de faible épaisseur, de manière à avoir l'incidence la plus faible possible sur l'épaisseur hors tout de la poche. Il doit cependant avoir un pouvoir filtrant et une capacité d'absorption des odeurs suffisants pour assurer au porteur d'une poche munie d'un tel filtre une autonomie allant d'un minimum de quelques heures à une durée aussi longue que 12 heures, 24 heures et même plus si possible; s'il s'agit d'un filtre incorporé à la poche lors de la fabrication de celle-ci, il doit en tout état de cause conserver toute son efficacité aussi longtemps que l'appareillage demeure en place. Pourtant les utilisateurs réclament de plus en plus des poches avec filtre pré-incorporé; c'est surtout le cas chez les patients agés, qui n'ont pas l'habileté requise pour effectuer eux-mêmes la pose et la mise en service, souvent délicates, de filtres séparés. Quel que soit le type des filtres pour poches de drainage actuellement sur le marché, ceux-ci n'ont en règle générale qu'une autonomie limitée à 12 heures au maximum, le plus souvent à 6 heures et même à quelques heures seulement pour les émissions de gaz particulièrement malodorants. On peut considérer ces durées de fonctionnement comme insuffisantes, notamment quand il s'agit d'appareillages vidangeables, puisque le patient est alors contraint de changer un appareillage dont la partie collectrice est pourtant encore utilisable.

On a maintenant conçu et élaboré un système de filtre pour poches de drainage, notamment pour colostomisés et pour iléostomisés, qui ne présente plus les défauts susdits et dont le mode de réalisation, tout en restant extrêmement simple, permet Leur totale incorporation auxdites poches,procure le moyen d'ajuster la durée de vie efficace dudit système de filtre de façon qu'elle corresponde au moins à la durée d'utilisation prévue de la poche et permet à l'utilisateur de déterminer lui-meme à convenance le débit à donner à ce système de filtre. L'ensemble de ces avantages,ainsi que d'autres, ressortira mieux de la description qui suit,où sont exposées les grandes lignes et des variantes de la présente invention et qui est compétée par les dessins annexés, qui illustrent plus concrètement certaines variantes.

L'invention a pour objet une poche de drainage d'anus artificiel comportant un système de filtre et d'évent, qui estcomposé d'une cartoche de matière filtrante pour les gaz située au niveau de la soudure des parois de cette poche,et constitue un filtre à passage direct, et d'une chambre aval délimitée uniquement par des lignes de soudure ou de collage des deux films formant les faces de la poche et par le filtre la paroi extérieure de la chambre pourant   être perforée à la fabrication ou par l'utilisateur pour obtenir un évent ladite poche étant caractérisée en ce que le filtre est séparé de l'intérieur de la poche par une chambre amont qui communique avec l'intérieur de la poche par une zone d'entrée, de dimensions Méduites et est délimitée par des lignes de soudure ou collage entre les deux filtres qui constituent les parois de la poche.

Pour la mise en oeuvre de la présente invention, le filtre qui constitue la base du système de filtre susdit est avantageusement composé d'une cartouche filtrante parallélépipédique, cylindrique ou autre. Pour simplifier l'exposé, l'invention sera décrite ci-après en référence à une cartouche filtrante parallélépipédique, mais il est clair que toute autre forme de filtre appropriée conviendrait également, pour autant qu'elle soit compatible avec les autres éléments du système de filtre.

La matière de filtrage constituant la partie active du filtre peut être granuleuse, fibreuse ou pulvérulente; une matière convenant tout particulièrement est par exemple constituée de granules de charbon actif ou d'une mousse à cellules ouvertes, imprégnée d'une substance efficace pour retenir les odeurs, telle que du charbon actif ou tout autre composé chimique approprié.

La cartouche de matière filtrante peut être directement emprisonnée entre les deux films de la poche et retenue par deux de ses côtés parallèles par soudure ou tout autre moyen de liaison des deux films de la poche entre eux le long desdits côtés.

En variante, la cartouche de matière filtrante peut comprendre, sur l'une ou l'autre de ses deux faces principales ou les deux, un film imperméable aux gaz, qui isole et retient la masse filtrante sur laquelle il est collé (ou fixé de manière étanche par tout autre moyen) de telle sorte que le système de filtre peut alors être rendu indépendant des films de la poche ou de l'un d'eux seulement, selon les cas, excepté le long des lignes de soudure ou de collage qui font intervenir les films de la poche.

Dans une forme de mise en oeuvre préférée, la cartouche, munie ou non de tels films imperméables aux gaz supplémentaires comporte sur au moins l'une de ses faces principales un adhésif apte à assurer un contact étroit et étanche aux gaz entre la cartouche et le film concerné de la poche ou de la cartouche.

Selon une autre variante, la cartouche filtrante peut être située pour partie à l'intérieur du volume normal de la poche et pour partie à l'extérieur de celui-ci et la fermeture du volume utile de la poche se fait alors, selon une ligne qui traverse les faces principales de la cartouche filtrante, par soudure ou collage de chacun des films de la poche sur la face principale concernée de ladite cartouche, le long d'une ligne dont les extrémités coïncident avec la ligne normale de soudure des deux films de la poche entre eux.

Dans le présent texte, les lignes de soudure ou de collage peuvent être des lignes droites, des lignes courbes ou même des lignes brisées.

Par ailleurs, selon l'invention, l'une des lignes de fixation de la cartouche de filtre aux films attenants est prolongée d'une certaine longueur de façon à définir un volume particulier, isolé du reste de la poche à l'exception d'une zone d'entrée de la cartouche filtrante et définissant en arrière de celle-ci (dans le sens de l'écoulement des gaz au travers de la cartouche filtrante) une chambre dite "chambre amont" destinée à réduire les risques de colmatage de la section d'entrée du filtre en maintenant relativement éloignées de celle-ci par ce moyen les matières liquides et solides que peut contenir ladite poche.

A son extrémité opposée, la cartouche de matière filtrante est mise en communication avec le milieu extérieur. Selon l'invention, on prévoit de faire déboucher la section de sortie de la cartouche dans un volume clos, défini par les deux films de la poche ou par le ou les films rapportés sur les faces de la cartouche filtrante, soudés ou collés ensemble de manière appropriée et dénommée ciaprès "chambre aval". Cette chambre aval peut être comprise dans le volume de la poche elle-même telle qu'elle aurait été réalisée en l'absence du système selon l'invention. Mais, de préférence, le volume de cette chambre aval déborde vers l'extérieur audelà de la ligne-enveloppe définissant la périphérie de la poche; de cette façon au moins une partie du volume de la chambre aval devient plus aisément repérable par l'utilisateur, qui peut alors plus facilement pincer entre deux doigts ce volume et y pratiquer la (ou les) perforation(s) qu'il lui incombe d'effectuer, quand et comme il le juge nécessaire, pour ouvrir un évent adapté à son cas particulier d'utilisation et de débit de gaz intestinaux. Il est clair qu'avec un tel dispositif la tâche de l'utilisateur se trouve grandement facilitée, alors même que le confort et la tranquillité que ce dispositif permet d'assurer sont parall•élément aussi fortement réhaussés. En variante, cette chambre aval peut être pré-perforée.

Ce système peut comporter en outre, si on le souhaite, un organe adhésif amovible et sensible à la pression, par exemple une languette adhésive, sur au moins l'un des films de la poche dans la portion de ceux-ci qui constitue la chambre aval, de façon à permettre l'obturation temporaire des perforations réalisées en dessous de cet organe adhésif, après décollement au moins partiel de celui-ci.

Dans un système de filtre conforme à la présente invention, la cartouche filtrante peut avoir une épaisseur ou un diamètre n'excédant pas 3 à 4 mm environ, tandis que sa longueur peut être quelconque, mais avantageusement supérieure à 12 mm et pouvant atteindre 50 mm ou plus. De par sa conception même, ce filtre permet une ventilation désodorisée des poches de drainage d'anus artificiel d'une durée et d'une efficacité notablement supérieures à ce que permettent les systèmes de filtre qui sont actuellement sur le marché. Ce système est aussi porteur d'autres avantages, dont certains ont été signalés plus haut et dont d'autres apparaitront à la lecture de la description qui suit.

L'invention est décrite ci-après plus concrètement en référence aux figures des planches de dessins, ci-annexées qui l'illustrent mais ne la limitent aucunement, et dans lesquelles:

- la figure 1 est une coupe schématique partielle d'un mode de réalisation du système de filtre et d'évent selon la présente invention,

- la figure 2 est une coupe schématique partielle d'un autre mode de réalisation du système selon l'invention,

- la figure 3 est une coupe schématique partielle d'un autre mode de réalisation du système de filtre et d'évent selon l'invention,

- la figure 4 est une coupe schématique partielle d'un autre mode de réalisation du système selon l'invention,

- la figure 5 est une coupe schématique partielle d'un autre mode de réalisation du système selon l'invention.

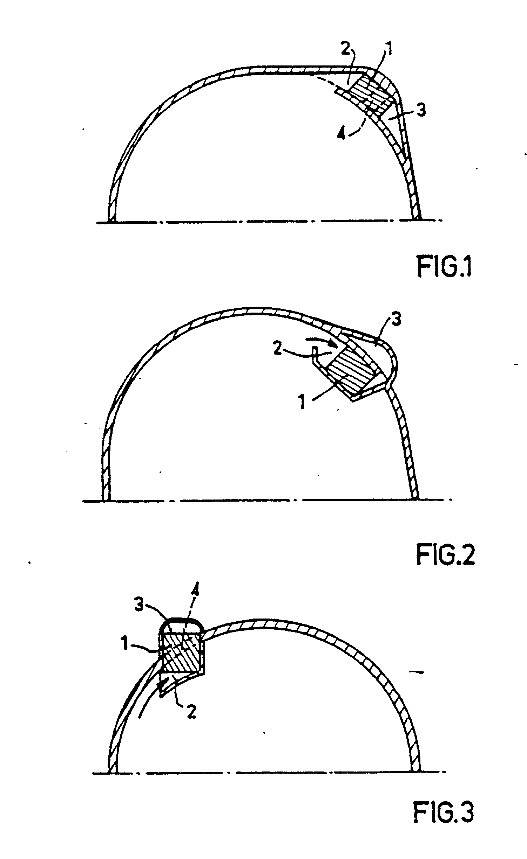

La figure 1 représente schématiquement la partie supérieure d'une poche de drainage,dans laquelle se trouve disposé un filtre 1, entre deux soudures des films de la poche qui se prolongent pour définir une chambre amont 2 et une chambre aval 3. En option,une zone ou une ligne de collage 4 peut venir parfaire le contact et donc l'étanchéité entre chacun des films de la poche et la surface correspondante de la cartouche filtrante. Le filtre est situé à l'extérieur de la ligne-enveloppe définissant le volume de la poche de base.

La figure 2 représente la partie supérieure d'une poche selon l'invention,dans laquelle les numéros de référence désignent les memes éléments que dans la figure 1. La cartouche filtrante est située à l'intérieur de la ligne-enveloppe définissant le contour de la poche;dans ce cas, on peut préférer rendre la cartouche indépendante d'au moins l'un des films constitutifs de la poche de recueil,par interposition d'un ou de deux films d'une part collé(s) sur la face correspondante du filtre et d'autre part collé(s) ou soudé(s) au(x) film(s) constituant la poche de recueil suivant une ligne correspondant à la soudure des films de la poche ou à l'extérieur de cette ligne.

La figure 3 représente une autre variante d'une partie supérieure de poche de drainage selon L'invention,dans laquelle la masse filtrante 1 est emprisonnée (contre-collée) entre deux films plastiques qui,débordants,sont soudés l'un à l'autre le long de chacun des côtés de la cartouche filtrante correspondant à la plus grande longueur de celleci et suivant le contour des chambres amont 2 et aval 3.

Une zone de collage ou de soudure supplémentaire 4 entre chacun des films plastiques de la cartouche filtrante et le film de la poche qui lui correspond vient parfaire l'étanchéité de l'ensemble,suivant la ligne enveloppe définissant le contour de la poche ou à l'extérieur de cette ligne.

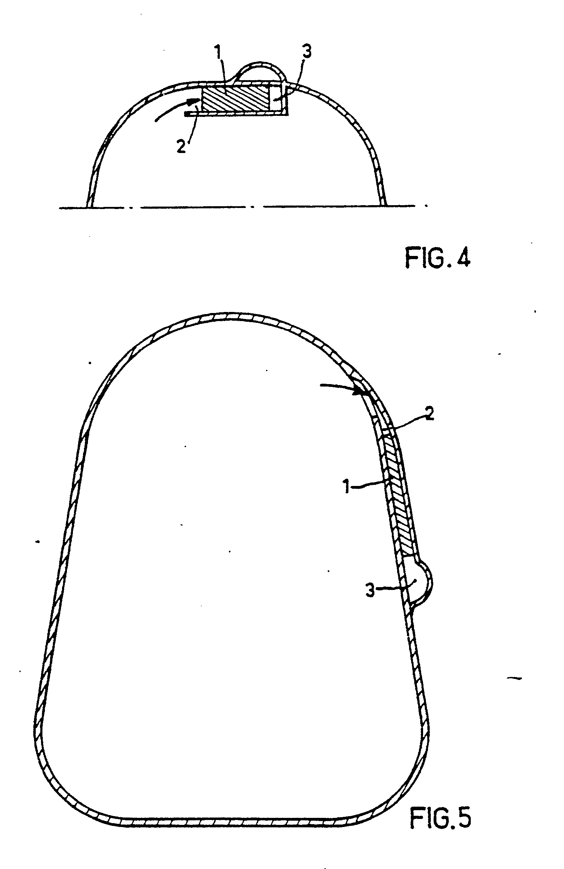

La figure 4 représente une variante du système selon la figure 2,les numéros de référence des divers éléments de ce système ayant là encore les memes significations.

La figure 5 représente un autre mode de réalisation de la variante selon la figure 1. Les proportions retenues empechent toute fuite périphérique,même sans collage additionnel de la masse filtrante contre les films,et autorisent l'utilisation de densités de masse filtrante moins élevées, d'où une meilleure longévité avec une perte de charge convenable,bien que la section soit réduite.

Dans chacun des modes de réalisation representés par les figures 1 à 5,l'ouverture de l'évent se fait,à la convenance de l'utilisateur,par ouverture du dégagement de perforations dans la chambre aval,dans laquelle aboutissent les gaz filtrés et désodorisés.

**Revendications**

1.Poche de drainage d'anus artificiel comportant un système de filtre et d'évent,composé d'une cartouche (1) de matière filtrante pour les gaz, située au niveau de la soudure des parois de la poche, et constituant un filtre à passage direct et d'une chambre aval (3) délimitée uniquement par des lignes de soudure ou de collage entre les deux films formant les faces de la poche et par le filtre (1) la paroi extêrieure de la chambre aval pourant être perforée à la fabrication ou par l'utilisateur, pour réaliser un évant, caractérisée en ce que le filtre est séparé de l'intérieur de la poche par une chambre amont (2) qui communique avec l'intérieur de la poche par une zone d'entrée,de dimensions réduites ladite chambre étant délimitée par des lignes de soudure ou collage entre les deux films qui constituent les parois de la poche.

2. Poche selon la revendication 1 caractérisée en ce que la cartouche filtrante (1) est parallélépipédique ou cylindrique.

3.Poche selon l'une des revendications 1 ou 2 caractérisée en ce que la cartouche filtrante (1) est directement emprisonnée entre les deux côtés de la poche et retenue par une soudure ou tout autre moyen de liaison entre les deux films formant les parois de la poche le long de ses deux côtés latéraux.

4.Poche selon l'une des revendications 1 à 3,caractérisée en ce que la cartouche filtrante (1) comprend sur l'une ou l'autre de ses faces latérales ou les deux,un film imperméable aux gaz qui isole et retient la masse filtrante sur laquelle il est collé ou fixé par tout autre moyen de maniere étanche.

5.Poche selon l'une des revendications 1 à 4,caractérisée en ce que la cartouche (1) est fixée par un adhésif à au moins un des films de la poche par l'un de ses côtés latéraux,de façon étanche aux gaz.

6. Poche selon l'une quelconque des revendications 1 à 5,caractérisée en ce qu'elle comporte en outre un organe adhésif,amovible, adhérent par pression au matériau de la poche,capable d'obturer la ou les perforations de la chambre aval (3)

## Patentansprüche

1. Ostomiebeutel für einen künstlichen Darmausgang, enthaltend ein Filter- und Entlüftungssystem, bestehend aus einer Patrone (1) aus Filtermaterial für die Gase, die auf gleicher Höhe der Schweißung der Beutelwände angeordnet ist und ein Filter zum direkten Durchlaß bildet, und aus einer nachströmseitigen Kammer (3), die allein durch die Schweiß- oder Klebenähte zwischen den beiden, die Seiten des Beutels bildenden Folien äußere Wand der Kammer bei der Herstellung oder durch den Benutzer perforiert werden kann, um eine Entlüftung zu erhalten, dadurch gekennzeichnet, daß das Filter gegenüber dem Inneren des Beutels durcheine vorströmseitige Kammer (2) getrennt ist, die mit dem Innern des Beutels über eine Eintrittszone mit verkleinerten Abmessungen kommuniziert, und daß die Kammer (2) durch die Schweiß- oder Klebenähte zwischen den beiden, die Wände des Beutels bildenden Folien begrenzt ist.

2. Beutel nach Anspruch 1, dadurch gekennzeichnet, daß die Filterpatrone (1) parallelepipedisch oder zylindrisch ausgebildet ist.

3. Beutel nach Anspruch 1 oder 2, dadurch gekennzeichnet, daß die Filterpatrone (1) unmittelbar zwischen den beiden Seiten des Beutels festgehalten und durch eine Schweißung oder andere Verbindungsmittel zwischen den beiden Folien, die die Wände des Beutels entlang seiner beiden Seiten bilden, gehalten ist.

4. Beutel nach einem der Ansprüche 1 bis 3, dadurch gekennzeichnet, daß die Filterpatrone (1) auf der einen oder anderen ihrer Seitenwände oder auf beiden Seitenwänden eine gasundurchlässige Folie enthält, die die Filtermasse isoliert und zurückhält, auf welcher sie aufgeklebt oder durch andere dichte Mittel befestigt ist.

5. Beutel nach einem der Ansprüche 1 bis 4, dadurch gekennzeichnet, daß die Filterpatrone (1) an einer ihrer seitlichen Seite mittels eines Klebers an wenigstens einer Folie des Beutels gasdicht befestigt ist.

6. Beutel nach einem der Ansprüche 1 bis 5, dadurch gekennzeichnet, daß er ferner ein abnehmbares Klebeorgan trägt, das durch Druck am Material des Beutels anhaftbar und fähig ist, die Perforation oder Perforationen der nachströmseitigen Kammer 3 zu verschließen.

## Claims

1. Drainage bag for an artificial anus, incorporating a filtering and venting system and composed of a cartridge (1) of filtering material for the gases, the cartridge being located at the region where the walls of the bag are welded and constituting a straight-through filter, and a downstream chamber (3) delimited exclusively by the welding lines or adhering lines between the two films which form the faces of the bag and by the filter (1), the outer wall of the said downstream chamber being able to be perforated on manufacture or by the user to produce a vent, characterized in that the filter is separated from the inside of the bag by an upstream chamber (2) which is connected to the inside of the bag through an admission zone of small size, the said chamber being delimited by the welding lines or adhering lines between the two films which constitute the walls of the bag.

2. Bag according to Claim 1, characterized in that the filtering cartridge (1) is parallelepipedal or cylindrical.

3. Bag according to one of Claims 1 or 2, characterized in that the filtering cartridge (1) is directly confined between the two sides of the bag and held in place by a weld or any other means of joining between the two films which form the walls of the bag along its two lateral sides.

4. Bag according to one of Claims 1 to 3, characterized in that the filtering cartridge (1) contains, on one or other or both of its lateral faces, a film which is impermeable to gases and which isolates and holds in place the filtering mass, to which it is adhered or attached by any other means in a leakproof manner.

5. Bag according to one of Claims 1 to 4, characterized in that the cartridge (1) is attached in a gastight manner by means of an adhesive to at least one of the films of the bag by one of its lateral sides.

6. Bag according to any one of Claims 1 to 5, characterized in that it incorporates in addition a removable adhesive member which adheres by pressure to the material of the bag and is capable of sealing the perforation or perforations in the downstream chamber (3).

FIG.1

FIG.2

FIG.3

FIG. 4

FIG.5